# EUROPÄISCHE PATENTSCHRIFT

(11) **EP 2 944 291 B1**
(45) Veröffentlichungstag und Bekanntmachung des Hinweises auf die Patenterteilung: **23.11.2016**
(21) Anmeldenummer: 15158804.3
(22) Anmeldetag: 12.03.2015
(51) Int. Cl.: A61F 2/52, B29C 43/20, B29C 43/18, B29C 43/12

(54) **VERFAHREN ZUR HERSTELLUNG EINER BRUSTPROTHESE**
METHOD FOR MANUFACTURING A BREAST PROSTHESIS
PROCÉDÉ DE FABRICATION D'UNE PROTHÈSE MAMMAIRE

(30) Priorität: 30.04.2014 DE 102014006313
(43) Veröffentlichungstag der Anmeldung: 18.11.2015
(73) Patentinhaber: Amoena Medizin-Orthopädie-Technik GmbH, 83064 Raubling (DE)
(72) Erfinder: Stadler, Maximilian, 83134 Prutting (DE)
(74) Vertreter: Laufhütte, Dieter

(56) Entgegenhaltungen:
- EP-A2- 0 880 951
- EP-A2- 1 857 080
- WO-A1-2010/015075

## Beschreibung

Die Erfindung betrifft ein Verfahren zur Herstellung einer Brustprothese, die im Wesentlichen aus in Kunststofffolien eingeschweißten, der Brustform nachgebildeten Körpern besteht.

Es sind bereits Brustprothesen bekannt, bei denen diese der Brustform nachgebildeten Körper aus einer transparenten additionsvernetzenden Zweikomponenten-Silikonkautschuk-Masse bestehen. Nachdem das Silikonmaterial von Natur aus transparent ist, wird die gewünschte Hautfarbe durch Zugabe von geringen Anteilen von Farbpigmenten - beispielsweise einer Zumischung von 0,2 Gewichtsprozent Farbpigment - erreicht. Typischerweise werden, als das Silikon umschließende Folien, Polyurethanfolien eingesetzt. Diese weisen eine hohe Elastizität und Weichheit auf. Dennoch sind sie im Vergleich zu dem verwendeten Silikon eher steif, so dass die Folie zur Faltenbildung neigt. Aufgrund der Lichtdurchlässigkeit der Polyurethanfolie und des Silikons sind diese Falten aber nicht deutlich sichtbar und stören deshalb nicht.

Durch Hinzugabe von Hohlkugelfüllstoffen, die zur Gewichtsreduzierung der Brustprothesen eingesetzt werden, ergibt sich eine stark deckende weiße Farbe in dem Leichtsilikon. Dies rührt daher, dass die Hohlkugelfüllstoffe aus einer Kugelvielzahl bestehen, die einzeln als Farbprisma wirken. Diese Erscheinung ist beispielsweise mit transparenten Schneekristallen vergleichbar, die aufgrund der Lichtbrechung in ihrer Summe ebenfalls weiß aussehen. Die weiße Farbe des Leichtsilikons kann durch die Zugabe von Farbpigmenten in Richtung einer Ausgleichung an die Hautfarbe verändert werden. Dies wird aber nicht so natürlich wiedergegeben, wie dies bei Standardsilikon möglich ist. Aufgrund dieser farblichen Unterschiede werden die zuvor erwähnten Falten der Polyurethanfolie durch die deckende Farbe des Leichtsilikons wesentlich stärker sichtbar und werden optisch als störend empfunden.

Zur Vermeidung des vorgenannten Problems ist es bereits bekannt geworden, eine mehrschichtige Brustprothese zu schaffen, wobei außen eine dünne Deckschicht aus Standardsilikon auf der Vorderseite gebildet wird, welches transparent ist und mit der an die Hautfarbe angepassten Farbgebung ein attraktives Erscheinungsbild ergibt. Aus der EP 0 880 951 B1 ist ein Verfahren zur Herstellung einer Mehrkammerbrustprothese bekannt, bei dem die Herstellung dieser sogenannten Kosmetikschicht außerhalb der Prothesenform erfolgt. Nachteilig bei diesem vorbekannten Verfahren ist es, dass die außerhalb der Prothesenform hergestellte Kosmetikschicht Dickenunterschiede zeigt, d.h. keine gleichmäßig dünne Außenschicht aufweist. Hierdurch wird das attraktive Erscheinungsbild, das durch die vergleichsweise dünne Deckschicht erreicht werden soll, häufig nur unzureichend erzielt.

Aufgabe der vorliegenden Erfindung ist es nun ein Verfahren zu entwickeln, dass eine derartige Prothese mit einer sogenannten kosmetischen Deckschicht mit konstanter Dicke schafft.

Erfindungsgemäß wird diese Aufgabe durch ein Verfahren nach dem Anspruch 1 gelöst.

Das erfindungsgemäße Verfahren zur Herstellung der Brustprothese, die im Wesentlichen ja aus in Kunststofffolien eingeschweißten der Brustform nachgebildeten Körper besteht, besteht aus folgenden Schritten:
- Schweißen eines Folienbeutels aus mindestens drei Folienlagen zur Erzeugung von mindestens zwei Kammern,
- Einlegen und Fixieren des Folienbeutels in das Prothesenformwerkzeug,
- Befüllen der außen liegenden Kammer mit einer vorzugsweise bereits bei Umgebungstemperatur oder bei leicht erhöhter Temperatur vernetzenden Masse,
- Vorvernetzen der Masse, während gleichzeitig die mindestens zweite Kammer mittels Luft oder einem anderen Fluid aufgeblasen wird,
- Ablassen der Luft oder des anderen Fluides aus der zweiten Kammer und anschließendes Befüllen der mindestens zweiten Kammer mit einer zweiten Masse,
- Schließen des Prothesenformwerkzeuges, um bei erhöhter Temperatur die in den Kammern befindlichen Massen vollständig zu vernetzen.

Entsprechend der Erfindung wird die Brustprothese mit einer sehr gleichmäßig dünnen kosmetischen Deckschicht durch den im vorgenannten Verfahren enthaltenen Vorvernetzungsschritt und durch gleichzeitiges Aufblasen der zweiten Kammer bzw. durch entsprechendes Befüllen mit einem anderen Fluid der zweiten Kammer hergestellt. Durch das vorherige Befüllen der ersten Kammer, die später die kosmetische Deckschicht bildet, sowie Vorvernetzen und das gleichzeitige Aufblasen der benachbarten zweiten Kammer innerhalb des Prothesenformwerkzeugs kann sichergestellt werden, dass sich eine gleichmäßige dünne Deckschicht auf der Vorderseite der Brustprothese ausbildet.

Sobald die in die erste Kammer eingefüllte Masse vorvernetzt ist, kann das in die zweite Kammer eingefüllte Gas bzw. Fluid abgelassen werden und diese Kammer kann mit der entsprechenden Masse befüllt werden, die üblicherweise neben dem vernetzenden Material zusätzlich Leichtfüllstoffe aufweist. Nach Schließen des Prothesenformwerkzeuges wird dieses in einem entsprechenden Ofen soweit aufgeheizt, dass die endgültige Formgebung der Brustprothese durch entsprechende Vernetzung der in die Kammern eingefüllten Masse und Verstreckung der Folie erfolgt.

Vorteilhafte Ausgestaltungen der Erfindung ergeben sich aus den sich an den Hauptanspruch anschließenden Unteransprüchen.

Demnach kann der Folienbeutel aus vier Folienlagen unter Bildung von drei Kammern gefertigt werden, wobei die vergleichsweise dünne dritte Kammer auf der Rückseite der Brustprothese angeordnet ist und die sogenannte Höhlungsschicht bildet, die an der Trägerin anliegt.

Besonders vorteilhaft wird die dritte Kammer zusammen mit der ersten Kammer mit einem Material gefüllt, dass zunächst ebenfalls nur vorvernetzt wird.

Die erste und gegebenenfalls die dritte Kammer können eine dünne äußere Schicht mit einer Schichtdicke von vorzugsweise 2 mm - 10 mm bilden, die die zweite Kammer ein- bzw. gegebenenfalls beidseitig umgeben.

Die den Folienbeutel bildenden Folienlagen können vorteilhaft aus Polyurethan bestehen. Die Polyurethanfolien können dabei eine Dicke von ca. 40 µm - 100 µm aufweisen.

Die Polyurethanfolien können mittels Thermoschweißen, HF-Schweißen (Hochfrequenzschweißen) oder Laserschweißen verschweißt werden. Die in den Kunststofffolien eingeschweißten Körper können aus einem additionsvernetzenden Silikon, einem thermoplastischen Polyurethan und/oder einem thermoplastischen Elastomer bestehen.

Die in der ersten Kammer des Folienbeutels eingefüllte erste Masse kann aus einer Silikonmischung bestehen, die schon bei Raumtemperatur hinreichend stark vernetzt, um nach wenigen Minuten Reaktionszeit weitgehend formstabil vorvernetzt zu sein.

Vorteilhaft wird die Temperatur zur vollständigen Vernetzung der in der zweiten Kammer des Folienbeutels eingefüllten Masse so gewählt, dass das Folienmaterial dauerhaft plastisch verformt wird, ohne dabei die Folie dauerhaft zu beschädigen. Hierdurch erfolgen eine Verstreckung der Folie und ein formgenaues Anlegen an die Oberfläche des Prothesenformwerkzeuges.

Gemäß einer weiteren besonders vorteilhaften Ausgestaltung der Erfindung werden die den Folienbeutel bildenden Folienlagen unter Bildung einer gleichmäßig breiten Schweißnaht verschweißt, wobei über die Wahl der Breite der Schweißnaht und die Wahl des Aufblasdrucks der zweiten Kammer die Gleichmäßigkeit der Schichtdicke einstellbar ist.

Vorteilhaft wird die Schweißnahtbreite der die erste Kammer bildenden Folien so breit gewählt, dass bei gegebenem Aufblasdruck eine gleichmäßige Schichtdicke gebildet wird, während die Schweißnaht der die dritte Kammer bildenden Folien vergleichsweise schmaler gebildet ist, um bei gleichem Aufblasdruck eine über die Kammer ungleichmäßige Schichtdicke derart zu erhalten, dass die Schicht zu der Schweißnaht dünner ausläuft, während sie zu Mitte hin dicker ist. Hierdurch kann in besonders vorteilhafter Weise eine erste kosmetische Deckschicht gebildet werden, die eine gleichmäßige Schichtdicke und daher eine gute optische Wirkung aufweist. Dagegen kann die dritte Kammer, die die Höhlungsschicht bildet in der Mitte zur besseren Druckverteilung dick ausgebildet sein, während sie zu den Rändern hin dünn ausläuft. Hierdurch schmiegt sich die Höhlungsschicht optimal an die Trägerin an.

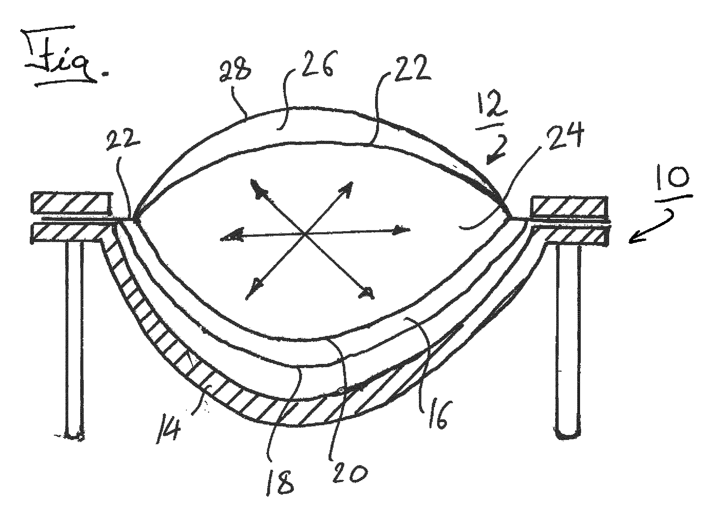

Weitere Merkmale, Einzelheiten und Vorteile der Erfindung werden anhand eines in der Zeichnung dargestellten Ausführungsbeispiels erläutert. Die einzige Figur zeigt eine schematische Darstellung eines hier nur teilweise dargestellten Prothesenformwerkzeuges, in welches eine Prothese zum Zeitpunkt der Vorvernetzung der Außenschichten eingelegt ist.

Bei der Herstellung der erfindungsgemäßen Brustprothese wird zunächst ein Folienbeutel aus mindestens drei Folienlagen geschweißt. Soweit drei Folienlagen verwendet werden, werden zwei Kammern gebildet. Alternativ können aber, wie in der Figur dargestellt, auch vier Folienlagen miteinander verschweißt werden, so dass drei Kammern entstehen. Die verwendete Folie besteht aus Polyurethan in einer Stärke von typischerweise 50 µm - 100 µm. Die Verschweißung der Folienlagen erfolgt durch Thermoschweißen, HF-Schweißen oder Laserschweißen. Der insgesamt mit 12 bezeichnete Folienbeutel wird in ein Prothesenformwerkzeug 10 eingelegt. Das Prothesenformwerkzeug 10 ist in der Figur nur teilweise dargestellt. Hier ist insbesondere der untere Formteil 14 gezeigt, der die Form der nachzubildenden Brust angibt. Das Gegenstück der Form, welches auf das Formteil 14 aufgesetzt wird, ist hier nicht im Einzelnen dargestellt. Hier ist lediglich der entsprechende Rand der Gegenform gezeigt.

Die einzelnen Folien des Folienbeutels sind anfänglich nicht vollständig auf Umfang abgeschweißt, so dass hier noch eine in der Figur nicht näher dargestellte Eingangsöffnung zum Einfüllen der im hier dargestellten Ausführungsbeispiel Silikonmischung vorhanden ist. Über die Öffnung wird zunächst die erste Kammer befüllt, die mit 16 bezeichnet ist und eine ca. 2 mm - 10 mm dicke kosmetische Deckschicht bildet.

Die diese erste Kammer 16 bildenden Folienlagen 18 und 20 werden unter Bildung einer gleichmäßig oder variabel breiten Schweißnaht 22 verschweißt. Nachdem die erste Kammer 16 mit einer additionsvernetzenden Zweikomponenten-Silikonkautschuk-Masse befüllt ist, wird diese entweder bei Umgebungstemperatur oder unter Zuführung von Wärme vorvernetzt. Während dieses Vorvernetzungsschrittes wird die sich an die erste Kammer 16 anschließende zweite Kammer 24 im hier dargestellten Ausführungsbeispiel mit Luft aufgeblasen, was durch die Doppelpfeile in der Figur dargestellt ist. Hierdurch wird also ein Druck auf die Folie 20 und die gegenüberliegende Folie 22 ausgeübt. Sowohl die Schweißnaht 22 wie auch der Aufblasdruck der in die zweite Kammer 24 eingepressten Luft führt zu einer gleichmäßigen Verteilung der Schichtdicke in der Kammer 16 während der Vorvernetzung der sich vernetzenden Masse. Über die Wahl der Breite der Schweißnaht und die Wahl des Aufblasdrucks kann ein optimiertes Ergebnis hinsichtlich der Gleichmäßigkeit der Schichtdicke erzielt werden. Im hier dargestellten Ausführungsbeispiel ist noch eine dritte Kammer 26 auf der Rückseite der zweiten Kammer 24 vorgesehen. Diese Kammer liegt auf der dem Körper zugewandten Seite. Diese dritte Kammer wird zusammen mit der ersten, äußeren Kammer 16 gefüllt und zusammen mit dieser vorvernetzt, während die zweite Kammer 24 entsprechend aufgeblasen wird. Die dritte Kammer 26, die durch die Folien 22 und 28 gebildet wird, weist im Gegensatz zur ersten Kammer 16 eine ungleichmäßige Schichtdicke auf. Hier soll gerade der mittlere Bereich zur besseren Druckverteilung am Körper dick ausgebildet sein, während die dritte Kammer zu den Rändern hin dünn auslaufen soll. Dies wird dadurch erreicht, dass im Bereich der Schweißnaht zwischen den Folien 22 und 28 gerade keine breite Schweißnaht ausgebildet wird, sondern dadurch, dass die Folien 22 und 28 hier unmittelbar spitz zulaufend miteinander verschweißt sind, wie dies in der Figur dargestellt ist.

Nach entsprechendem Vorvernetzen der in der ersten Kammer 16 und der dritten Kammer 26 befindlichen vernetzenden Masse wird die in der zweiten Kammer 24 unter Überdruck gehaltene Luft abgelassen. Anschließend wird die zweite Kammer 24, die die Hauptkammer darstellt, befüllt. Hier wird ein Leichtsilikon, also eine Mischung aus Silikon und Hohlkugelfüllstoff im vorliegenden Ausführungsbeispiel eingefüllt. Anschließend wird die zweite hier nicht mehr dargestellte Formhälfte des Prothesenformwerkzeugs aufgesetzt. Diese Formhälfte ist so gestaltet, dass die aus den drei Kammern bestehende Prothese umschlossen ist. Die entsprechend geschlossenen Form wird anschließend in einen entsprechenden Ofen gestellt, in dem die Temperatur sich vornehmlich nach der Erweichungstemperatur und den Erweichungszeiten der den Beutel bildenden Polyurethanfolie gewählt wird. Die Temperatur ist so eingestellt, dass bei der Polyurethanfolie eine dauerhafte, plastische Verformung erreicht wird, ohne dass die Folie jedoch thermisch beschädigt wird. Üblicherweise werden hier Temperaturen von ca. 130° C eingestellt. Bei dieser Temperatur vernetzen die vernetzenden Materialien in den einzelnen Kammern vollständig.

Nach diesem Fertigungsschritt wird die Prothese aus der Form genommen und die überstehende Folie beispielsweise mit einem scharfen Messer, einer Schere oder einem Stanzwerkzeug abgetrennt.

## Patentansprüche

1. Verfahren zur Herstellung einer Brustprothese, die im wesentlichen aus in Kunststofffolien eingeschweißten, der Brustform nachgebildeten Körpern, vorzugsweise aus einer transparenten additionsvernetzenden Zweikomponenten-Silikonkautschuk-Masse, besteht, mit folgenden Schritten:
- Schweißen eines Folienbeutels aus mindestens drei Folienlagen zur Erzeugung von mindestens zwei Kammern,
- Einlegen und Fixieren des Folienbeutels in das Prothesenformwerkzeug
- Befüllen der außen liegenden Kammer mit einer vorzugsweise bereits bei Umgebungstemperatur oder bei leicht erhöhter Temperatur vernetzenden Masse,
- Vorvernetzen der Masse, während gleichzeitig die mindestens zweite Kammer mittels Luft oder einem anderen Fluid aufgeblasen wird,
- Ablassen der Luft oder des anderen Fluids aus der zweiten Kammer und anschließendes Befüllen der mindestens zweiten Kammer mit einer zweiten Masse,
- Schließen des Prothesenformwerkzeuges, um bei erhöhter Temperatur die in den Kammern befindlichen Massen vollständig zu vernetzen.

2. Verfahren nach Anspruch 1, **dadurch gekennzeichnet, dass** der Folienbeutel aus vier Folienlagen unter Bildung von drei Kammern gefertigt wird, wobei die vergleichsweise dünne dritte Kammer auf der Rückseite der Brustprothese angeordnet ist.

3. Verfahren nach Anspruch 1, **dadurch gekennzeichnet, dass** die dritte Kammer zusammen mit der ersten Kammer mit einem Material gefüllt wird, das zunächst ebenfalls nur vorvernetzt, wird.

4. Verfahren nach einem der vorangehenden Ansprüche, **dadurch gekennzeichnet, dass** die erste und gegebenenfalls die dritte Kammer eine dünnen äußere Schicht mit einer Schichtdicke von vorzugsweise 2-10 mm bilden, die die zweite Kammer ein- bzw. gegebenenfalls beidseitig umgeben.

5. Verfahren nach einem der vorangehenden Ansprüche, **dadurch gekennzeichnet, dass** die den Folienbeutel bildenden Folienlagen aus Polyurethan bestehen.

6. Verfahren nach Anspruch 5, **dadurch gekennzeichnet, dass** die Polyurethanfolien ein Dicke von 40 µm - 100 µm aufweisen.

7. Verfahren nach einem der Ansprüche 1 bis 6, **dadurch gekennzeichnet, dass** die Polyurethanfolien mittels Thermoschweißen, HF-Schweißen oder Laserschweißen verschweißt werden.

8. Verfahren nach einem der Ansprüche 1 bis 7, **dadurch gekennzeichnet, dass** die in den Kunststofffolien eingeschweißten Körper aus einem additionsvernetzenden Silikon, einem thermoplastischen Polyurethan und/oder einem thermoplastischen Elastomer bestehen.

9. Verfahren nach einem der Ansprüche 1 bis 8, **dadurch gekennzeichnet, dass** die in der ersten Kammer des Folienbeutels eingefüllte erste Masse aus einer Silikonmischung besteht, die schon bei Raumtemperatur hinreichend stark vernetzt, um nach wenigen Minuten Reaktionszeit weitgehend formstabil zu sein.

10. Verfahren nach einem der Ansprüche 1 bis 9, **dadurch gekennzeichnet, dass** zur vollständigen Vernetzung der in der zweiten Kammer des Folienbeutels eingefüllten Masse die Temperatur so gewählt wird, dass das Folienmaterial dauerhaft plastisch verformt wird, ohne dabei die Folie dauerhaft zu beschädigen.

11. Verfahren nach einem der Ansprüche 1 bis 10, **dadurch gekennzeichnet, dass** die den Folienbeutel bildenden Folienlagen unter Bildung einer gleichmäßig breiten Schweißnaht verschweißt werden, wobei über die Wahl der Breite der Schweißnaht und die Wahl des Aufblasdrucks die Gleichmäßigkeit der Schichtdicke einstellbar ist.

12. Verfahren nach Anspruch 11, **dadurch gekennzeichnet, dass** die Schweißnahtbreite der die erste Kammer bildenden Folien so breit gewählt ist, dass bei gegebenem Aufblasdruck eine gleichmäßige Schichtdicke gebildet wird, während die Schweißnaht der die dritte Kammer bildenden Folien vergleichsweise schmaler gebildet ist, um bei gleichem Aufblasdruck eine über die Kammer ungleichmäßige Schichtdicke derart zu erhalten, dass die Schicht zu der Schweißnaht dünner ausläuft, während sie zur Mitte hin dicker ist.

## Claims

1. A method for manufacturing a breast prosthesis which substantially consists of bodies modeling the shape of the breast, which are shrink-wrapped in plastic films, preferably of a transparent, addition cross-linking two-component silicone rubber compound, comprising the following steps:
- welding of a film bag from at least three film layers for producing at least two chambers,
- inserting and fixing the film bag in the prosthesis molding tool,
- filling of the outer chamber with a compound preferably crosslinking already at ambient temperature or at slightly elevated temperature,
- pre-crosslinking of the compound, while at the same time the at least second chamber is inflated by means of air or another fluid,
- draining of the air or the other fluid from the second chamber, and subsequently filling the at least second chamber with a second compound,
- closing of the prosthesis molding tool, in order to completely cross-link the compounds present in the chambers at elevated temperature.

2. The method according to claim 1, **characterized in that** the film bag is fabricated from four film layers by forming three chambers, wherein the comparatively thin third chamber is arranged on the back of the breast prosthesis.

3. The method according to claim 1, **characterized in that** the third chamber together with the first chamber is filled with a material which initially likewise is only pre-crosslinked.

4. The method according to any of the preceding claims, **characterized in that** the first and possibly the third chamber form a thin outer layer with a layer thickness of preferably 2 mm - 10 mm, which surround the second chamber on one side and possibly on two sides.

5. The method according to any of the preceding claims, **characterized in that** the film layers forming the film bag are made of polyurethane.

6. The method according to claim 5, **characterized in that** the polyurethane films have a thickness of 40 µm - 100 µm.

7. The method according to any of claims 1 to 6, **characterized in that** the polyurethane films are welded together by means of thermal welding, HF welding or laser welding.

8. The method according to any of claims 1 to 7, **characterized in that** the bodies shrink-wrapped in the plastic films are made of an addition cross-linking silicone, a thermoplastic polyurethane and/or a thermoplastic elastomer.

9. The method according to any of claims 1 to 8, **characterized in that** the first compound filled into the first chamber of the film bag consists of a silicone mixture which cross-links sufficiently strongly already at room temperature, in order to be largely dimensionally stable after few minutes of reaction time.

10. The method according to any of claims 1 to 9, **characterized in that** for the complete cross-linkage of the compound filled into the second chamber of the film bag the temperature is chosen such that the film material permanently is plastically deformed without permanently damaging the film.

11. The method according to any of claims 1 to 10, **characterized in that** the film layers forming the film bag are welded together by forming a welding seam of uniform width, wherein by choosing the width of the welding seam and choosing the inflation pressure the uniformity of the layer thickness is adjustable.

12. The method according to claim 11, **characterized in that** the welding seam width of the films forming the first chamber is chosen so broad that at a given inflation pressure a uniform layer thickness is formed, while the welding seam of the films forming the third chamber is chosen comparatively narrower, in order to obtain a layer thickness non-uniform across the chamber at the same inflation pressure such that the layer thins out towards the welding seam, while it is thicker towards the center.

## Revendications

1. Procédé de fabrication d'une prothèse mammaire qui est constituée sensiblement par des corps soudés dans des feuilles de matière plastique et imitant la forme du sein, de préférence par une masse de caoutchouc silicone à deux composants transparente et réticulante par addition, présentant les étapes suivantes :
- soudage d'un sac en feuilles constitué par au moins trois couches de feuilles pour produire au moins deux chambres,
- insertion et fixation du sac en feuilles dans l'outil de formage de prothèse,
- remplissage de la chambre située à l'extérieur avec une masse qui réticule de préférence déjà à température ambiante ou à une température légèrement élevée,
- préréticulation de la masse tandis qu'en même temps, la deuxième chambre est gonflée au moyen d'air ou d'un autre fluide,
- évacuation de l'air ou de l'autre fluide hors de la deuxième chambre et remplissage consécutif de ladite au moins deuxième chambre avec une deuxième masse,
- fermeture de l'outil de formage de prothèse pour réticuler entièrement la masse se trouvant dans les chambres à température élevée.

2. Procédé selon la revendication 1, **caractérisé en ce que** le sac en feuilles est réalisé à partir de quatre couches de feuilles en formant trois chambres, la troisième chambre comparativement mince étant agencée sur la face arrière de la prothèse mammaire.

3. Procédé selon la revendication 1, **caractérisé en ce que** la troisième chambre, conjointement avec la première chambre est remplie avec un matériau qui n'est tout d'abord également que préréticulé.

4. Procédé selon l'une quelconque des revendications précédentes, **caractérisé en ce que** la première et, le cas échéant, la troisième chambre forment une couche extérieure mince avec une épaisseur de couche de préférence de 2 à 10 mm, qui entoure la deuxième chambre d'un côté ou, le cas échant, des deux côtés.

5. Procédé selon l'une quelconque des revendications précédentes, **caractérisé en ce que** les couches de feuilles formant le sac en feuilles sont en polyuréthane.

6. Procédé selon la revendication 5, **caractérisé en ce que** les feuilles de polyuréthane ont une épaisseur de 40 µm à 100 µm.

7. Procédé selon l'une quelconque des revendications 1 à 6, **caractérisé en ce que** les feuilles de polyuréthane sont soudées par thermosoudage, par soudage à haute fréquence, ou par soudage au laser.

8. Procédé selon l'une quelconque des revendications 1 à 7, **caractérisé en ce que** les corps soudés dans les feuilles de matière plastique sont constituées par un silicone réticulant par addition, un polyuréthane thermoplastique et/ou un élastomère thermoplastique.

9. Procédé selon l'une quelconque des revendications 1 à 8, **caractérisé en ce que** la première masse remplie dans la première chambre du sac en feuilles est constituée par un mélange de silicone qui est réticulé de manière suffisamment forte déjà à température ambiante pour être dimensionnellement stable dans une large mesure après quelques minutes de temps de réaction.

10. Procédé selon l'une quelconque des revendications 1 à 9, **caractérisé en ce que** pour la réticulation totale de la masse remplie dans la deuxième chambre du sac en feuilles, la température est choisie telle que le matériau de feuille est durablement soumis à une déformation plastique sans que la feuille soit durablement endommagée.

11. Procédé selon l'une quelconque des revendications 1 à 10, **caractérisé en ce que** les couches de feuille formant le sac en feuilles sont soudées en formant une ligne de soudure de largeur régulière, la régularité de l'épaisseur de couche étant réglable par le choix de la largeur de la ligne de soudure et par le choix de la pression de gonflage.

12. Procédé selon la revendication 11, **caractérisé en ce que** la largeur de ligne de soudure des feuilles formant la première chambre est choisie telle que dans le cas d'une pression de gonflage donnée, une épaisseur de couche régulière est formée tandis que la ligne de soudure des feuilles formant la troisième chambre est formée de manière comparativement plus étroite pour obtenir, avec la même pression de gonflage, une épaisseur de couche inégale sur la chambre, de telle sorte que la couche devient plus mince vers la ligne de soudure alors qu'elle est plus épaisse vers le milieu.
